(19) Europäisches Patentamt / European Patent Office / Office européen des brevets

(11) **EP 2 217 928 B1**

(12) **EUROPEAN PATENT SPECIFICATION**

(45) Date of publication and mention
of the grant of the patent:
**09.08.2017 Bulletin 2017/32**

(21) Application number: **08856043.8**

(22) Date of filing: **03.12.2008**

(51) Int Cl.:
*G01N 33/573* (2006.01)  *G01N 33/53* (2006.01)
*G01N 33/535* (2006.01)  *G01N 33/542* (2006.01)
*G01N 33/58* (2006.01)  *B01L 3/00* (2006.01)
*C12Q 1/68* (2006.01)  *G01N 33/543* (2006.01)
*C08G 83/00* (2006.01)

(86) International application number:
**PCT/US2008/013363**

(87) International publication number:
**WO 2009/073201 (11.06.2009 Gazette 2009/24)**

(54) **ALTERNATE LABELING STRATEGIES FOR SINGLE MOLECULE SEQUENCING**

ALTERNATIVE MARKIERUNGSVERFAHREN FÜR EINZELMOLEKÜLSEQUENZIERUNG

STRATÉGIES DE MARQUAGE ALTERNÉES POUR SÉQUENÇAGE DE MOLÉCULE UNIQUE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **04.12.2007 US 5407 P**

(43) Date of publication of application:
**18.08.2010 Bulletin 2010/33**

(73) Proprietor: **Pacific Biosciences of California, Inc.**
Menlo Park, CA 94025 (US)

(72) Inventors:
• **KORLACH, Jonas**
Newark, CA 94560 (US)
• **ROITMAN, Daniel**
Menlo Park, CA 94025 (US)
• **EID, John**
San Francisco, CA 94103 (US)
• **OTTO, Geoff**
Brookline, MA 02446 (US)
• **HARDENBOL, Paul**
San Francisco, CA 94110 (US)
• **FLUSBERG, Benjamin**
Palo Alto, CA 94306 (US)

(74) Representative: **Kiddle, Simon John et al**
Mewburn Ellis LLP
City Tower
40 Basinghall Street
London EC2V 5DE (GB)

(56) References cited:
WO-A1-03/048769      WO-A2-03/014743
WO-A2-03/064608      WO-A2-2006/044078
WO-A2-2007/075873    US-A- 6 027 946
US-A1- 2005 266 456  US-A1- 2005 266 456
US-A1- 2007 003 949  US-A1- 2007 009 980
US-B1- 7 181 122     US-B2- 6 607 878

• **EID JOHN ET AL: "Real-time DNA sequencing
from single polymerase molecules.", SCIENCE
(NEW YORK, N.Y.) 2 JAN 2009 LNKD-
PUBMED:19023044, vol. 323, no. 5910, 2 January
2009 (2009-01-02), pages 133-138, XP002613182,
ISSN: 1095-9203**
• **STRIEBEL H-M ET AL: "Enhancing sensitivity of
human herpes virus diagnosis with DNA
microarrays using dendrimers", EXPERIMENTAL
AND MOLECULAR PATHOLOGY, ACADEMIC
PRESS, US, vol. 77, no. 2, 1 October 2004
(2004-10-01), pages 89-97, XP004537435, ISSN:
0014-4800, DOI:
DOI:10.1016/J.YEXMP.2004.05.004**
• **JONAS KORLACH ET AL: "Selective aluminum
passivation for targeted immobilization of single
DNA polymerase molecules in zero-mode
waveguide nanostructures", PROCEEDINGS OF
THE NATIONAL ACADEMY OF SCIENCES,
NATIONAL ACADEMY OF SCIENCES, US, vol.
105, no. 4, 29 January 2008 (2008-01-29), pages
1176-1181, XP002632441, ISSN: 0027-8424, DOI:
10.1073/PNAS.0710982105 [retrieved on
2008-01-23]**

• JONAS KORLACH ET AL: "Long, Processive Enzymatic DNA Synthesis Using 100% Dye-Labeled Terminal Phosphate-Linked Nucleotides", NUCLEOSIDES, NUCLEOTIDES AND NUCLEIC ACIDS, TAYLOR & FRANCIS, PHILADELPHIA, PA, USA, vol. 27, no. 9, 1 September 2008 (2008-09-01), pages 1072-1082, XP008144483, ISSN: 1525-7770, DOI: 10.1080/15257770802260741 [retrieved on 2008-08-18]

**Description**

**FIELD OF THE INVENTION**

The invention relates to systems and methods providing multi-ligand constructs and labeling strategies, including fluorescence based, non-fluorescence based, and non-optical based labels, e.g., for use with single molecule sequencing.

**BACKGROUND OF THE INVENTION**

Fluorescence is a primary detection means in numerous areas of molecular biology. Fluorescence is typically a detection means of choice because it is highly sensitive and permits detection of single molecules in a variety of assays, including, e.g., nucleic acid sequencing, amplification and hybridization. Single molecule detection can be performed using pico to nanomolar concentrations of fluorophore for individual molecule detection, or extremely small observation volumes can be used to detect individual molecules up to, e.g., micromolar reagent concentrations. For example, "zero-mode waveguides" (ZMWs), constructed from arrays of subwavelength holes in metal films can be used to reduce the observation volume of a sample of interest for single molecule detection during processes such as single molecule nucleic acid sequencing. *See,* e.g., Levene, et al. (2003) Zero-Mode Waveguides for Single Molecule Analysis at High Concentrations" Science 299:682-686.

Although fluorescence is sensitive enough to provide for single molecule detection, there are certain disadvantages to its use in particular settings. For example, the detection of a fluorophore is typically limited by the quantum yield of that particular fluorophore. Additionally, the presence of autofluorescence in a sample being analyzed and in the detection optics of the relevant detection system can be problematic, particularly in epifluorescent application. The lack of photostability of fluorophores, and photodamage effects of excitation light on an analyte or reactant of interest can also cause problems. The cost of the relevant analysis system is also an issue due to, for example, the need for high energy excitation light sources.

A variety of approaches have been taken to improve fluorescent detection limits and reduce the costs associated with the associated analysis systems. These include optimization of detection system optics, use of enhancers to increase quantum yield, etc. For example, excitation light can be reflected through a sample multiple times to improve quantum yield without increasing the output of the excitation source (*see,* e.g., Pinkel, *et al.*, SPECIMEN ILLUMINATION APPARATUS WITH OPTICAL CAVITY FOR DARK FIELD ILLUMINATION, U.S. Pat. No. 5,982,534). Fluorescent emissions that occur in a direction other than towards detection optics can also be redirected towards the optics, thereby improving the percentage of emission photons detected by the system (*see,* e.g., White, *et al.*, SIGNAL ENHANCEMENT FOR FLUORESCENCE MICROSCOPY, U.S. Pat. No. 6,169,289). Quantum yield enhancers such as silver particles have also been used to enhance fluorescence in samples (reviewed in Aslan, et al., 2005, "Metal-enhanced fluorescence: an emerging tool in biotechnology," Current Opinion in Biotechnology 16:55-62). Yield enhancers can result in detection of intrinsic fluorescence of certain molecules such as DNA even without the use of fluorescent labels (*see* Lakowicz, et al., 2001, "Intrinsic Fluorescence from DNA Can Be Enhanced by Metallic Particles," Biochemical and Biophysical Research Communications, 286:875-879).

WO 2006/044078 relates to arrays of optical confinements suitable for single molecule analysis. Eid et al. (Science, 323: 133-138, 2009) describes real-time DNA sequencing using single polymerase molecules in zero-mode waveguides. Striebel et al. (Exp. Mol. Path., 77: 87-97, 2004) describes the use of dendrimers to enhance the sensitivity of DNA microarrays for herpes virus detection. WO 03/014743 relates to the use of dendrimers to enhance fluorescent signals in arrays.

Notwithstanding such other approaches, additional compositions and methods that enhance fluorescence detection or even replace fluorescence detection with other routes of detection are highly desirable and will allow development of new applications that rely on such improved detection methods. Additionally, ligand compositions that provide multiple ligands and/or multiple labels per construct would increase the probability of the ligand successfully interacting with the enzyme, decrease the concentration of construct provided per detection volume (while maintaining the higher ligand concentration in the assay). Furthermore, smaller, multiply-labeled multi-ligand constructs will fit more easily within, e.g., the ZMW detection zone typically employed in SMRT™ sequencing, thereby increasing the signal-to-noise ratio of nucleotide incorporation events and decreasing the background signal, as well as increasing the rate of successful incorporations and decreasing the rate of missed incorporations. The present application provides these and other features that will be apparent upon complete review of the following.

**SUMMARY OF THE INVENTION**

The present invention provides methods, and systems for monitoring an enzymatic reaction between an enzyme and a ligand. Optionally the detecting step involves non-optically detecting the labeled construct, e.g., using a non-optical sensor that is functionally coupled to the substrate surface.

In one aspect, the present invention provides a method of monitoring a single molecule real-time enzymatic reaction between an enzyme and a member ligand of a plurality of ligands, the method comprising:

providing a substrate comprising a substrate surface, a zero mode waveguide (ZMW) detection volume proximal to the substrate surface, and a single molecule of an enzyme positioned within the ZMW detection volume and bound to or associated with the substrate surface, wherein the enzyme comprises a polymerase;

contacting the enzyme with a detectable construct comprising a labelled DNA dendrimer or a labelled circular DNA framework, wherein multiple ligands specific for the enzyme are removably coupled to the framework and wherein the ligands comprise one or more nucleotides or nucleotide analogs;

detecting the construct while the enzyme and a member ligand of the multiple ligands are interacting, thereby monitoring the enzymatic reaction.

In a further aspect, the present invention provide a system for monitoring a single molecule real-time enzymatic reaction, the system comprising:

a substrate comprising a substrate surface and a ZMW detection volume proximal to the substrate surface;

a single molecule of an enzyme, which enzyme is positioned within the ZMW detection volume and bound to or associated with the substrate surface, wherein the enzyme comprises a polymerase;

a detectable construct comprising a labelled DNA dendrimer or a labelled circular DNA framework, wherein multiple ligands specific for the enzyme are removably coupled to the framework and wherein the ligands comprise one or more nucleotides or nucleotide analogs; and

a detector functionally coupled to the substrate surface and capable of detecting the construct when the construct is in proximity of the enzyme.

The present disclosure comprises methods of monitoring enzymatic reactions through tracking light occlusion and/or light scattering. In such methods a substrate surface is provided, along with an enzyme that is bound to or associated with the substrate surface (which can optionally comprise, e.g., a surface in a zero mode waveguide). Such methods also entail providing one or more ligands that comprise an occluding and/or light scattering moiety and that are specific for the enzyme; interacting the enzyme and the ligands (e.g., under reaction conditions appropriate for the reaction to proceed); and monitoring light transmission past or through the substrate surface and/or monitoring light scattering away from the substrate surface. In such methods, the ligands can comprise, e.g., four different ligands that are each labeled with a different occluding and/or light scattering moiety. For example, the ligands can comprise four different nucleotides and/or nucleotide analogues, while the enzyme can comprise a nucleic acid polymerase. Also, in such methods, the occluding and/or light scattering moiety can comprise, e.g., a metal nanoparticle, a plastic nanoparticle, a glass nanoparticle, or a semiconductor material nanoparticle. Thus, in particular embodiments, as each different ligand interacts with the enzyme (e.g., as when a polymerase incorporates a nucleotide into a growing oligonucleotide), the progress can be monitored through detection of the different light occluding/scattering that is associated with each particular ligand.

In other aspects, the disclosure comprises systems for monitoring enzymatic reactions through tracking light occlusion and/or light scattering. Such systems can comprise a substrate surface (which can optionally comprise, e.g., a surface in a zero mode waveguide), an enzyme (e.g., a nucleic acid polymerase) that is bound to or associated with the substrate surface; one or more ligands that are specific for the enzyme and which each comprise an occluding and/or light scattering moiety, a light source, and a detection component for detecting light transmission past or through the substrate surface and/or for detecting light scattering away from the substrate surface. In such systems, the ligands can optionally comprise, e.g., four different nucleotide and/or nucleotide analogues (each labeled with a different light occluding and/or light scattering molecule) and the enzyme can comprise a nucleic acid polymerase. The occluding and/or light scattering moiety can comprise, e.g., a metal nanoparticle, a plastic nanoparticle, a glass nanoparticle, or a semiconductor material nanoparticle.

Methods and systems for monitoring a single molecule real-time enzymatic reaction between an enzyme and a member ligand of a plurality of ligands are also provided. The systems include, but are not limited to a substrate having a substrate surface and a detection volume proximal to the substrate surface; an enzyme which is positioned within the detection volume and bound to or associated with the substrate surface; a detectable construct; and a detector functionally coupled to the substrate surface and capable of detecting the labeled construct when the construct is in proximity of the enzyme. As disclosed herein, the detectable construct comprises a labelled DNA dendrimer or a labelled circular DNA framework. In alternative embodiments, the framework comprises a metal particle, a magnetic particle, or a light occluding/scattering particle as provided herein.

The methods for monitoring single molecule real-time enzymatic reactions using the multi-ligand constructs of the claimed invention include the steps of providing a substrate comprising a substrate surface, a detection volume proximal to the substrate surface, and a single molecule of an enzyme positioned within the detection volume and bound to or associated with the substrate surface. A detectable construct comprising a detectable framework and a plurality of ligands specific for the enzyme is provided; the construct is then detected while interacting the enzyme and a member ligand (the ligands being removably coupled to the framework), thereby monitoring the enzymatic reaction.

In other aspects, the disclosure comprises methods of monitoring enzymatic reactions through tracking fluorescence wherein multiple ligands (e.g., multiple copies of the same ligand) are associated with a single fluorescent particle. Such methods can comprise: providing a substrate surface (e.g., a substrate within a zero mode waveguide); providing an enzyme (such as nucleic acid polymerase) that is bound to or associated with the substrate surface; providing one or more ligands that are specific for the enzyme, wherein each ligand is bound to a fluorescent particle and wherein at least two ligands are bound to each fluorescent particle; interacting the enzyme and the ligands (e.g., under reaction conditions appropriate for the reaction to proceed); providing a excitation light source; and monitoring a change in the fluorescence of the ligand(s). For example, the ligands can comprise four different nucleotides and/or nucleotide analogues, while the enzyme can comprise a nucleic acid polymerase. Thus, in particular embodiments, as each different ligand interacts with the enzyme (e.g., as when a polymerase incorporates a nucleotide into a growing oligonucleotide), the progress can be monitored through detection of different fluorescences that are associated with each particular ligand (e.g., due to a different dye moiety being associated with each different ligand). In some embodiments, each fluorescent particle is only (or is substantially only) associated with or bound to a single type of ligand (e.g., one fluorescent particle bound to multiple copies of a single type of nucleotide). The fluorescent particles can comprise, e.g., a quantum dot, nanoparticle, or nanobead.

In some aspects, the disclosure comprises systems for monitoring enzymatic reactions through tracking fluorescence wherein multiple ligands (e.g., multiple copies of the same ligand) are associated with a single fluorescent particle. Such systems can comprise: a substrate surface (e.g., a surface within a zero mode waveguide); an enzyme (e.g., a nucleic acid polymerase) that is bound to or associated with the substrate surface; one or more ligands that are specific for the enzyme, wherein each ligand is bound to a fluorescent particle and wherein at least two ligands (e.g., two copies of the same ligand) are bound to each fluorescent particle; an excitation light source; and a detection component for detecting changes in fluorescence of the fluorescent particle. In particle embodiments, the ligands can comprise four different nucleotides and/or nucleotide analogues, while the enzyme can comprise a nucleic acid polymerase. Thus, in particular embodiments, as each different ligand interacts with the enzyme (e.g., as when a polymerase incorporates a nucleotide into a growing oligonucleotide), the progress can be monitored through detection of different fluorescences that are associated with each particular ligand. In particular embodiments of such systems, substantially no ligands of any ligand type are attached to a fluorescent particle having a ligand of any other ligand type (i.e., each fluorescent particle is only associated with or bound to a single type of ligand). In some embodiments, the fluorescent particle comprises a quantum dot, nanoparticle, or nanobead.

These and other objects and features of the invention will become more fully apparent when the following detailed description is read in conjunction with the accompanying figures. The invention is defined by the claims.

## BRIEF DESCRIPTION OF THE DRAWINGS

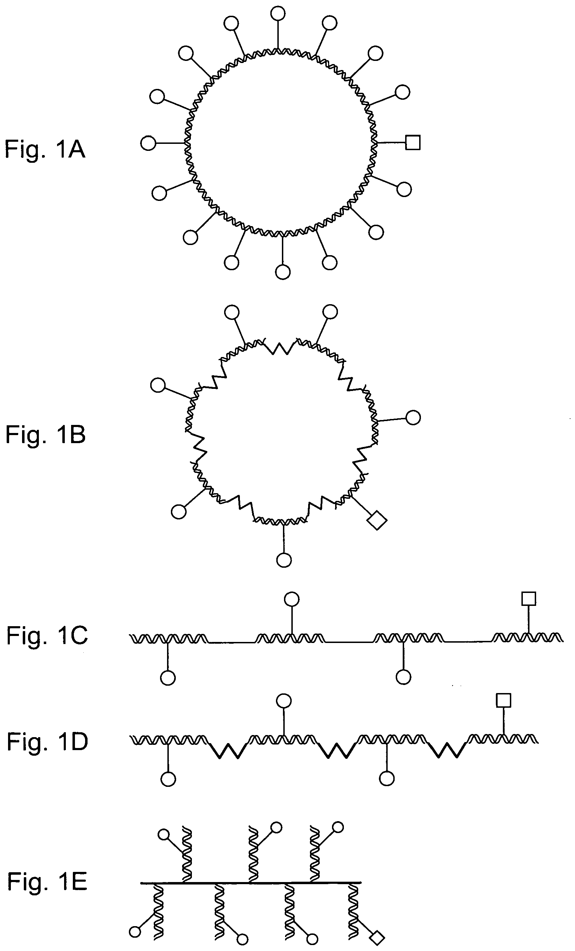

Figure 1, panels A through E, provides various embodiments of the nucleic acid-based frameworks of the invention.

## DETAILED DESCRIPTION

The present disclosure provides a variety of systems and methods for enhancing fluorescent analyte signal strength and detection of fluorescent analyte signals, as well as systems and methods for enhancement and detection of analyte signals other than fluorescence. The features of the invention are particularly useful for the detection of low copy number analytes, e.g., for single molecule detection. This type of detection is useful to reduce reagent consumption for e.g., DNA sequencing reactions, and for the detection of rare analytes, as well as to reduce detection system costs by reducing the amount of illumination light required for detection.

Several approaches are used to achieve enhanced signal production and detection in the embodiments herein. For ease of presentation, the approaches are divided into fluorescence-based approaches and non-fluorescence based approaches. Of course, it will be appreciated that such categorization should not necessarily be taken as limiting and that particular strategies can combine elements of both approaches. Additionally, the various embodiments are optionally used in any combination with one another and/or with additional approaches not recited herein.

In the fluorescence-based approaches, the invention comprises a number of embodiments. For example, the invention comprises methods and systems for analyte monitoring (e.g., single molecule sequencing using ZMWs) through fluorescence polarization to aid in differentiation between signals associated with true nucleotide incorporation events and other transient optical signal events. Additionally, the disclosure comprises methods and systems using Lanthanide dyes, where time gated FRET is detected for analyte monitoring. The disclosure also comprises methods and systems which use terminal phosphate-mediated multiple nucleotide fluorescent particle complexes as well as embodiments comprising energy conductive polymers and embodiments comprising three-dye, four-color sequencing strategies.

In the non-fluorescence based approaches herein, the disclosure includes embodiments that monitor change in optical properties other than fluorescence, such as optical occlusion or light scattering, to monitor analyte reactions (again, e.g., single molecule sequencing optionally using ZMWs). Other embodiments include systems and methods to monitor changes in electrical and/or magnetic properties that are associated with analyte reactions, e.g., through use of giant magnetoresistance sensing.

Furthermore, the disclosure includes compositions (as well as related methods and systems) that provide multiple ligands and/or bear multiple labels per construct. The construct typically have a detectable framework to which a plurality of ligands are removably coupled. In some embodiments, the detectable framework is a metal particle, magnetic particle, or light occluding/scattering particle; in other embodiments, the framework further includes one or more labels (fluorescent or otherwise) for detection purposes.

SINGLE MOLECULE DETECTION

While the various embodiments herein are primarily discussed in terms of their application to single molecule sequencing (and primarily in regard to sequencing with use of ZMWs) it will be appreciated that the methods and systems are also applicable for use with monitoring of other enzymatic systems, e.g., immunoassays, drug screening, and the like, and/or in non-confined detection systems, e.g., systems which do not use ZMW or similar confinement schemes.

The detection of activity of a single molecule of enzyme, or of a few proximal molecules, as with particular embodiments of the instant invention, has a number of applications. For example, single molecule detection in sequencing applications can be used to monitor processive incorporation of nucleotides by polymerases while avoiding issues of de-phasing among different complexes. Such de-phasing can be a deficiency of various approaches based on multi-molecule monitoring of populations. The embodiments of the present invention can increase effective readlength, which effectively increases sequencing throughput. Similarly, monitoring of individual complexes through the invention provides direct readout of reaction progress. Such direct readout is superior to the average based information obtained from bulk assays. Detection of single molecule activity or of low numbers of molecules can similarly be used to reduce reagent consumption in other enzymatic assays.

Single molecule monitoring or single analyte monitoring finds beneficial use in single molecule sequencing (the observation of template dependent, polymerase mediated primer extension reactions which are monitored to identify the rate or identity of nucleotide incorporation, and thus, sequence information). In particular, individual complexes of nucleic acid template, polymerase and primer are observed, as sequentially added nucleotides are incorporated in the primer extension reaction. The bases can include label moieties that are incorporated into the nascent strand and detected (thus indicating incorporation), but which are then cleaved away, resulting in a native DNA product that permits further extension reactions following washing steps. Alternatively and preferably, cleavage of the label group can occur during the incorporation reaction, e.g., through the use of nucleotide analogs labeled through the polyphosphate chain *(see,* e.g., U.S. Patent No. 6,399,335) which allows incorporation to be monitored in real time. In one particularly elegant approach, a polymerase reaction is isolated within an extremely small observation volume, effectively resulting in observation of individual polymerase molecules. In the incorporation event, observation of an incorporating nucleotide analog is readily distinguishable from non-incorporated nucleotide analogs based upon the distinguishable signal characteristics of an incorporating nucleotide as compared to randomly diffusing non-incorporated nucleotides. In a preferred aspect, such small observation volumes are provided by immobilizing the polymerase enzyme within an optical confinement, such as a Zero Mode Waveguide (ZMW). For a description of ZMWs and other optical confinements and their application in single molecule analyses, and particularly nucleic acid sequencing, *see,* e.g., Eid et al, "Real-Time DNA Sequencing from Single Polymerase Molecules," Science 20 November 2008 (10.1126/science.1162986), Levene, et al., "Zero-mode waveguides for single-molecule analysis at high concentrations," Science 299:682-686 (2003), U.S. Patent Nos. 6,917,726, 7,013,054, 7,033,764, 7,052,847, 7,056,661, 7,056,676, and 7,181,122, and Published U.S. Patent Application No. 2003/0044781. Because of the inherent limitation on detectability of single molecule approaches, novel and innovative labeling and/or detection schemes such as those of the present invention are useful in enhancing detection of such analytical reactions.

In particular aspects of single molecule monitoring, the analyte or ligand (e.g., a nucleotide analog) includes a label, e.g., a fluorescent label or a non-fluorescent label such as are described herein. The label is used to track the progress of an enzymatic reaction in single molecule analyses, e.g., in a ZMW or other device. Optionally, the ligand (or plurality of ligands) and the label (or plurality of labels) are associated with a framework structure, to form a detectable construct. As explained throughout, the label can comprise a fluorescent label or a non-fluorescent label. In either instance, the label can be associated with the analyte/ligand by any of a number of techniques known in the art, examples of such are given herein.

FLUORESCENCE BASED LABELING STRATEGIES

As stated previously, the embodiments are roughly divided into two groups - embodiments comprising fluorescence detection and embodiments comprising non-fluorescence detection. The embodiments having fluorescence detection comprise, e.g., methods of using fluorescence polarization to differentiate between background fluorescence noise and fluorescence indicating analyte activity, use of lanthanide labels, use of multi-ligand detectable constructs or multiple nucleotide complexes as labels, use of energy conductive polymers, and methods of using 3 dye / 4 color sequencing. All of such embodiments that use fluorescence are primarily described with respect to use with single molecule sequencing (especially using ZMWs), however, it will be appreciated that the teachings of the embodiments also encompass other applications such as monitoring product formation or use in different enzymatic reactions, etc.

SINGLE MOLECULE SEQUENCING WITH FLUORESCENCE POLARIZATION

The fluorescence observed from fluorescently labeled nucleotide analogs during single molecule sequencing (e.g., in ZMWs) is not restricted to only fluorescence from analogs that undergo incorporation into an extending polynucleotide. Additional fluorescence arises from, e.g., nonspecific sticking of dye to substrate or protein surfaces, branching fraction (i.e., non-incorporation interactions between nucleotide analogues and polymerase complexes), and non-cognate sampling, all of which add to general background noise contributions. Fluorescence intensity measurements alone sometimes cannot differentiate pulses due to such noise contributions from those due to actual incorporation of nucleotide analogs into an extending polynucleotide.

To help ameliorate such background fluorescence, the instant embodiment comprises the use of polarization information to allow differentiation between a true incorporation signal and other background fluorescence noise. Anisotropy can be used to detect rotational mobility both in bulk (*see,* e.g., Czeslik, et al., Biophys. J., 2003, 84:2533, and U.S. Patent No. 6,689,565 to Nikiforov), and at the single molecule level (*see* Dehong Hu and H. Peter Lu, J. Phys. Chem. B, 2003, 107:618). The current embodiment furthers use of polarization information, especially in regard to single molecule sequencing reactions.

The fluorescence anisotropy of a fluorophore emitter is dependent on its rotational diffusivity as well as on its excited state lifetime ($\tau$). The lifetime is, in turn, a report on the microenvironment of the dye. The basic equation covering fluorescence anisotropy is:

$$\text{(Equation 1)} \qquad \frac{r_0}{r} = 1 - \frac{\tau}{\theta} = 1 + 6D\tau$$

where D is the rotational diffusion coefficient. Furthermore, $\theta$ is defined as:

$$\text{(Equation 2)} \qquad \theta = \frac{\eta V}{RT}$$

where $\eta$ is the viscosity, V is the volume of the fluorophore system, R is the gas constant, and T is the temperature of the system. From the equations it can be seen that a fluorescent nucleotide analog that is sequestered in a polymerase active site can be differentiated from one that is freely diffusing by the restricted rotational mobility of the bound analog. Measurement of the anisotropy illustrates the increased signal to noise ratio because the diffusion background is selected against based on its comparatively low anisotropy.

Use of the anisotropy measurements in the current embodiment allows a distinction to be made between a fluorescent nucleotide analog that simply explores a polymerase active site (e.g., branching fraction) and one that actually continues on to incorporation into an extending polynucleotide with the concomitant release of a dye labeled cleavage product. In particular, by monitoring the ability of the dye moiety to emit depolarized fluorescence in response to polarized excitation light, one can monitor the rotational diffusion rate of the dye and, by implication, monitor different stages in an incorporation and/or non-incorporation signal event.

For example, when a fluorophore that is attached to the triphosphate end of a nucleotide analog is incorporated into a growing DNA strand during duplication on a surface-bound polymerase, there are two relevant events where the analysis of the emission polarization in the current embodiment improves the measurement. The first improved measurement location arises during the immobilization of the nucleotide-dye complex in the active site of the polymerase while the second occurs during release of the dye-pyrophosphate complex. During the first event, the emission anisotropy increases due to steric interactions of the analog with the polymerase that transiently limit the rotational diffusion of the analog during the incorporation event. This interaction momentarily reduces the rotational diffusion and consequently yields a reduction in depolarized fluorescence obtained from the dye. In the second event, the release of the dye-pyrophosphate following incorporation of the nucleotide portion results in an increase in the rotational diffusion of the free dye-pyrophosphate as compared to the dye-analog, and consequently, an increase in the depolarized fluorescence. Restated, the anisotropy undergoes a rapid decay below the base line because the dye pyrophosphate can undergo faster rotation than the dye-triphopshate analog.

While the difference in rotational diffusion between the cleavage product and the intact nucleotide analog provides a small signal, the excited state lifetime of the dye can be affected significantly by the release of the base. This directly impacts the observed anisotropy (*see* above equations). Moreover, the current embodiment also comprises monitoring of a single analog of sufficient sensitivity which allows system optimization with regard to finding conditions that maximize the ratio of incorporation to non-incorporation events.

Use of the current embodiment allows differentiation between signals that result from an incorporation event, signals that result from background presence of labeled nucleotides, non-incorporation interactions between analogs and polymerase complexes (also termed "branching fraction"), and signals that result from non-transient artifacts, such as non-specific dye "sticking" to substrate or protein surfaces.

The use of real time anisotropy information to elucidate the dye microenvironment depends on the achievable time resolution which is photon limited. For a count rate of 3 kHz, resolutions under 100 ms are achievable. Improvements to time resolution can be made by using a maximum likelihood estimator that remains robust even with as few as 50 photons. This yields a time resolution in the neighborhood of tens of milliseconds. Given that nucleotide analog residence times is in the 50-100 ms range, this achieves the necessary resolution. Additionally, the ZMW modality can yield an additional resolution factor by augmenting the fluorophore brightness. *See,* e.g., J. Wenger, et al. Optics Express, 2005, 13:7035.

The information gathered in use of this embodiment can be implemented both in the ZMW modality and in other excitation schemes, such as total internal reflection fluorescence (TIRF) based analytical schemes. The feasibility of use of single molecules in a TIRF scheme has been demonstrated in the literature. *See* Dehong Hu and H. Peter Lu, J. Phys. Chem. B, 2003, 107:618.

MULTI-LIGAND CONSTRUCTS

There are several disadvantages to monitoring ligand:enzyme interactions using simple constructs comprising an individual ligand labeled with a single fluorescent molecule: for example, the fluorescent signal may be weak or difficult to monitor, and incorporation events can be missed if the dye molecule is photobleached, photodamaged, or otherwise non-functional. Nucleic acid sequencing strategies such as SMRT™ sequencing would benefit from methods and systems that provide compositions that have more than one label per nucleotide. Furthermore, these same sequencing strategies would also benefit from techniques and compositions that enable or provide more than one nucleotide per fluorophore (or other detectable label).

To address these difficulties, a further embodiment provides detectable constructs bearing a plurality of ligands and/or a plurality of label moieties, as well as related methods and systems. The detectable constructs typically include a detectable framework and a plurality of ligands removably coupled to the framework (e.g., releasable upon interaction with the target enzyme).

For example, the detectable constructs can be used in methods of monitoring single molecule real-time enzymatic reactions between an enzyme and a member ligand of a plurality of ligands. The methods include providing a substrate having a substrate surface as well as a detection volume proximal to the substrate surface. A single molecule of an enzyme is bound to or associated with the substrate surface, such that the enzyme is positioned within the detection volume. After adding the construct to the reaction mixture, the construct is detected during the interaction between the enzyme and a member ligand of the plurality of ligands, thereby monitoring the enzymatic reaction.

Systems for monitoring an enzymatic reaction are also provided herein. The claimed systems include a substrate comprising a substrate surface and a detection volume proximal to the substrate surface; an enzyme positioned within the detection volume and bound to or associated with the substrate surface; the detectable construct as provided herein; and a detector functionally coupled to the substrate surface and capable of detecting the labeled construct when the construct is in proximity of the enzyme (e.g., during the interaction between the ligand and enzyme).

Those of skill in the art will appreciate that the numerous embodiments of the claimed multi-ligand constructs, methods and systems provided herein are exemplary.

Terminal phosphate mediated multiple nucleotide fluorescent particle complexes

As noted above, single molecule sequencing can benefit from high fluorescence signal to noise ratio in comparison of the incorporation signal relative to background diffusion. Additionally, single molecule sequencing can also benefit from little or slow enzyme branching during cognate incorporation. Branching is the rate of dissociation of a nucleotide or nucleotide analogue from the polymerase active site without incorporation of the nucleotide or nucleotide analogue where if the analogue were incorporated would correctly base-pair with a complementary nucleotide or nucleotide analogue in the template.

The current embodiment simultaneously addresses both of these concerns by use of a fluorescent particle:nucleotide complex. The structure of the complex includes a framework comprising a single, central fluorescent particle/nanobead/quantum dot. Multiple nucleotides (of identical base composition) are attached to this framework, typically by the terminal phosphate of the nucleotide. This complex yields an effectively "high" nucleotide concentration at a relatively "low" fluorescent molecule concentration. This, therefore, increases the relative signal to noise by decreasing the effective background fluorescence concentration while maintaining an identical nucleotide concentration. This complex can also aid in reduction of the branching fraction problem through the effective increase of the local concentration of the correct nucleotide due to rapid re-binding of the nucleotide-particle which masks the effects of the enzymatic branching.

Those of skill in the art will appreciate that the current embodiment is not limited by the nature of the framework (e.g., the central particle/bead/quantum dot). Attachment of nucleotides to various nanoparticles is well known those of skill. *See,* e.g., U.S. Patent Nos. 6,979,729; 6,387,626; and 6,136,962; and Published U.S. Patent Application No. 2004/0072231. Additionally, the nature of the fluorescent tag on the central particle can vary between embodiments, as can immobilization strategy of the terminal phosphate. Furthermore, the density of the immobilized nucleotide on the particle can also vary in different applications or within the same method (e.g., different nucleotides within the same reaction can optionally comprise different densities). In some instances, the embodiment utilizes polymerase enzymes that are specifically created/selected having desired kinetic properties, e.g., lower Km.

Optionally, the framework comprises more than one fluorescent moiety coupled to the central particle/bead/quantum dot. Details regarding embodiments comprising a plurality of labels (e.g., in conjunction with a plurality of ligands) is provided below.

Dendrimer Frameworks

In the methods and systems of the invention the detectable construct comprises a nucleic acid-based framework. For example, in some embodiments, the framework comprises a labeled DNA dendrimeric composition. DNA dendrimers are typically composed of one or more dendrimer monomer units. Each monomer has a central region of double-stranded DNA and four single-stranded arms. Dendrimeric structures can also be prepared using RNA, and by using alternative structural forms of nucleic acids (for example, Z-DNA or peptide nucleic acids).

Optionally, multiple copies of the monomer units can be linked together (e.g., via complementary binding of the single-stranded arms) to create a larger polymeric species having more than four single-stranded arms. One or more label moieties (e.g., fluorescent labels), ligands such as nucleotides, linker molecules, or other target molecules can be coupled to the dendrimeric monomer or polymer. Optionally, these ligand or label moieties are conjugated to the single-stranded arms of the dendrimer (e.g., those not involved in formation of the dendrimeric polymer) via, for example, complementary binding of the dendrimer arm to a nucleic acid (or peptide nucleic acid) sequence comprising the ligand or a portion thereof (e.g., a portion acting as a linker region). Alternatively, the ligand and/or label moieties are coupled to the double-stranded arm or body portion of a dendrimer unit.

Thus, dendrimer-based compositions can be used as frameworks and offer a simple approach to providing multiple labels and/or multiple ligands on a single detectable construct. An additional advantage of employing a DNA dendrimer as a framework for the labeled constructs of the invention is the composition's large negative charge, which may reduce or prevent indiscriminate adhesion of the construct to the substrate surface or other assay device components.

As noted above, the framework can comprises a single dendrimer monomer unit, or a plurality of dendrimer monomers hybridized to form a dendrimeric polymer (Nilsen et al. 1997 "Dendritic Nucleic Acid Structures" J. Theoretical Biology, 187:273-284; Wang et al. 1998 "Dendritic Nucleic Acid Probes for DNA Biosensors" JACS 120:8281-8282). The polymeric DNA dendrimers can be spherical, cylindrical, or have other shapes; the overall molecular weight and number of free arms available in the polymeric composition can readily be varied without undue experimentation. In addition, one of skill in the art would readily be able to generate and/or alter the length and/or composition (nucleic acid sequence) of either/both the arms and the body of the dendrimer monomer unit, e.g., in order to optimize the construct for use in a specific assay.

Dendrimeric compositions for use as frameworks in the detectable constructs, methods and systems of the disclosure are also commercially available. See, for example, the 3DNA dendrimer monomers available from Genisphere (Hatfield, PA; on the world wide web at genisphere.com).

Optionally, the ligands comprising the plurality of ligands are removably coupled to one or more single-stranded arms of the dendrimeric composition. The mechanism for associating the ligand with the dendrimer includes complementary binding between an available dendritic single stranded arm sequence and the ligand, or a DNA, RNA or PNA sequence (e.g., a linker) releasably coupled to the ligand.

While the labeled dendrimer-type constructs comprise at least one ligand and at least one detectable label, in a preferred embodiment, multiple detectable labels and/or multiple ligands (e.g., nucleotides) are attached to the dendrimer framework. In general, one would want to conjugate one or more nucleotides of a single type to a given species of dendrimeric construct. In addition, for purposes of detection, one would typically attach at least one, and preferably a plurality, of label moieties (albeit not necessarily of the same type) to that same dendrimer species. For SMRT™ sequencing, the nucleotide ligands are typically coupled to the dendrimer framework (preferably the dendrimeric arm or a linker moiety coupled thereto) via the nucleotides' gamma-phosphate.

Circular DNA frameworks

In an alternate embodiment, labeled circular nucleic acid species can also be used as frameworks in the systems and methods of the invention. Preferably, the labeled circular nucleic acid species is compact enough to fit in a selected detection volume proximal to the substrate surface.

In some embodiments, the circular nucleic acid framework comprises a double-stranded nucleic acid molecule. Exemplary double-stranded nucleic acid molecules for use as frameworks include, but are not limited to, double-stranded DNA molecules, duplexes of two peptide nucleic acid (PNA) molecules, and DNA:PNA hybrid duplexes. Use of PNA:PNA or PNA:DNA duplex constructs has the additional advantage of reducing the charge on the nucleic acid circle, potentially improving the polymerase's ability to incorporate nucleotides from the construct. Furthermore, RNA or Z-DNA can be used as the labeled circular nucleic acid species. Optionally, the circular nucleic acid molecule is shaped in a dumbbell-like structure, with a double-stranded portion in the middle, flanked by single-stranded loops.

While the labeled circular species comprises at least one ligand and at least one detectable label, in a preferred embodiment, multiple detectable labels and/or multiple ligands (e.g., nucleotides) are attached along the length of the circular nucleic acid framework. As noted above, releasable coupling of the nucleotide ligand can be achieved either directly, or via linker molecules attached to the DNA bases or their phosphate groups. In embodiments in which the detectable label comprises one or more fluorophores, the fluorophore labels are optionally spaced far enough apart from one another (e.g., at least 5 bases apart, at least 10 bases apart, at least 15 bases apart, or greater) so that quenching is prevented or minimized.

One preferred spatial arrangement of ligands along the circular nucleic acid construct is to spatially alternate the ligands with the labels. This arrangement increases the likelihood of ligand presentation and incorporation (by reducing an orientation bias of the circular detectable construct); in addition, such an arrangement would minimize quenching among fluorophore-type ligands. In an alternate preferred embodiment, the ligands are positioned on one portion, or "side" of the circular construct, and the labels are positioned on the opposite, distal side of the construct. In embodiments involving nucleotide ligands and fluorophore labels, separation of the ligands and fluorophores keeps the latter distal from the polymerase enzyme, thus reducing the potential for photo-induced damage of the polymerase. In a further preferred embodiment, the plurality of fluorophore ligands comprise more than one type of ligand; the two types of fluorophores are intentionally positioned close or proximal to one another (e.g., a few bases apart) to enable FRET. In the methods and systems that utilize such embodiments, a single laser line can potentially yield emission of, e.g., both green and red fluors.

In general, at least one ligand, and preferably a plurality of ligands of a single type are releasably coupled to a single circular construct. In addition, one would optionally attach at least one detectable label, and preferably a plurality of labels (e.g., fluorophores), but not necessarily all of the same type), to the circular nucleic acid construct. For methods and systems for SMRT™ sequencing, the circular construct is releasably coupled to the nucleotide ligands via the nucleotides' gamma-phosphate.

Other nucleic acid frameworks

In further embodiments the framework comprises a nucleic acid molecule (linear or circular) having multiple double-stranded sections interspersed with non-double-stranded linker regions (see Figure 1). Exemplary linker regions include, but are not limited to, portions of single stranded DNA and polyethylene glycol (PEG) molecules. Optionally, the one or more labels are coupled to the double-stranded sections of the detectable construct. In some embodiments, the nucleic acid framework is circular; alternatively, the nucleic acid framework is a linear dendrimer-like nucleic acid molecule, and preferably a DNA molecule, in which the linear double-stranded sections (with labels coupled thereto) fan out of a backbone structure such as a PEG linker.

Figure 1 provides depictions of various embodiments of the nucleic acid-based frameworks of the invention. A detectable construct comprising a circular double-stranded DNA framework bearing a plurality of attachments is depicted in Figure 1A. The tethered structures represent ligands or label moieties (or a combination thereof); the tethered squares (□) represent ligands (e.g., releasable nucleotides); the tethered dots (○) represent either ligands or labels (e.g., fluors). The number and relative ratio of labels and ligands can vary from those depicted. For example, each construct provided in Figure 1 bears at least one ligand and a plurality of additional attachment, which can be either additional ligands or label moieties.

In Figure 1B, a related embodiment of detectable construct is provided, in which the circular framework comprises alternating sections of double-stranded nucleic acid and linker regions (represented by the "sawtooth" regions). In the depicted embodiment, the ligands/labels are shown as attached to the double-stranded regions; however, they could also (or alternatively) be attached to the linker regions. The linker regions confer increased flexibility and, optionally, a reduction in size, to the constructs; exemplary linker moieties include, but are not limited to, polyethylene glycol (PEG).

Figure 1C through 1E provide depictions of linear framework moieties, in which the double-stranded nucleic acid portions are interspersed with either regions of single-stranded nucleic acid (Figure 1C) or linker moieties such as PEG (Figure 1D and 1E). In Figure 1E, a plurality of double-stranded nucleic acids (with associated ligand/labels) are coupled to a linear linker molecule to form a "branched" framework.

NON-FLUORESCENT LABELING STRATEGIES

As detailed previously, the present disclosure also presents non-emissive, e.g., non-fluorescent, labeling strategies. Such strategies provide advantages in situations where one or more of the excitation radiation, the fluorescent emissions, or the overall fluorescent chemistry may interfere with a given reaction to be monitored. For example, in some cases, it has been observed that the light sources utilized in monitoring/observation of various enzymatic activities with fluorescently labeled reactants (e.g., fluorescent nucleotides used in single molecule sequencing reactions) may have damaging effects on prolonged enzyme activity in the system.

The non-fluorescent labeling embodiments herein can be employed to overcome such concerns through use of non-fluorescent or even non-optical labeling of ligand moieties (e.g., nucleotide analogs in single molecule sequencing). While the non-fluorescent and non-optical embodiments herein are primarily discussed in terms of their application to single molecule sequencing (and primarily in regard to sequencing with use of ZMWs) it will be appreciated that the methods and systems are also applicable to use with other enzymatic systems, e.g., with immunoassays, enzyme activity analyses, receptor binding assays, drug screening assays, and the like, and/or in non-confined detection systems, e.g., in systems which do not use ZMW or similar confinement schemes.

ZMW OCCLUSION FOR SINGLE MOLECULE DNA SEQUENCING

As explained herein (*see also*, U.S. Pat. Nos. 6,917,726 to Levene et al. and 7,056,661 to Korlach et al.) typical variations of single molecule sequencing in ZMWs take advantage of the exponential decay of light in waveguide structures to observe very small reaction volumes that include individual polymerization complexes while masking out background concentrations of analytes. Thus, the goal or benefit of the system is not for light transmission to occur through the waveguide, but rather for non-propagating modes to exist in the waveguide. To monitor analyte/ligand activity, e.g., as in single molecule sequencing, the current embodiment, however, takes advantage of the extremely small amounts of light transmitted through waveguides.

As is well known in the art, light intensity through a zero mode waveguide decays exponentially. *See,* e.g., Heng, et al., 2006, "Characterization of light collection through a subwavelength aperture from a point source," Optics Express, 14(22): 10410-10425 for further discussion of light transmission. The instant embodiment utilizes opaque and/or light scattering nanoparticles as frameworks in light scattering/occluding (i.e., detectable) constructs to monitor real time polymerization inside ZMWs. The presence of the opaque or light scattering nanoparticle changes the transmission characteristics of the ZMW. Thus, a change in the properties of the space within a ZMW changes transmission or reflective properties of the waveguide and, therefore, allows detection of presence of particular analytes.

In the current embodiment, different nucleobases are differentiated by different opaque and/or light scattering nanoparticle frameworks bound or attached to the nucleotides (e.g., individually, or a plurality of nucleotide ligands). In embodiments comprising opaque nanoparticles, the different nanoparticles occlude the transmissivity of the waveguide to varying degrees to distinguish between nucleotides. In embodiments comprising light scattering nanoparticles, the different nucleotides are distinguished by the degree/amount of light scattering rather than the amount of transmissivity through the waveguide.

The physical characteristics of the various detectable constructs can be used to differentiate between the bases based on size (e.g., different constructs comprise differently sized nanoparticles which thus block/scatter different amounts of light) or by material (e.g., some nucleotides comprise opaque nanoparticles and others comprise light scattering nanoparticles). Detectable constructs of different sizes produce different magnitudes of diminution of the transmissivities of the ZMW. For example, occlusion of a 50 nm diameter ZMW by a 10 nm particle produces a different diminution than occlusion of the same diameter ZMW by a 40 nm particle, thereby allowing differentiation between the different nucleotides to which the particles are attached. In other embodiments, some nucleotides comprise opaque nanoparticles, while other comprise light scattering nanoparticles in order to differentiate between the different nucleotides. In yet other embodiments, nucleotides are differentiated based on degree/amount of light scattering from different light scattering moieties attached to different nucleotides.

In certain settings, the current embodiment is used without ZMWs. For example when the Km of the nucleotide analogs to the polymerase is very low, or when the scattering signal can be enhanced, e.g., by coupling into surface plasmons by a proximal metallic layer, then the embodiment optionally does not comprise use of a ZMW.

Other advantages of the current embodiment include reduction of potential problems of template accessibility at the ZMW bottom because layers thinner than 100 nm are more suitable for maximum signal to noise of occlusion.

Furthermore, in various permutations of the instant embodiment, ZMW cladding materials other than Al are optionally used, as the opaqueness of the cladding is less critical than for it is for embodiments comprising fluorescence confinement.

The opaque and light scattering nanoparticles of the embodiment can comprise one or more of a number of different materials. Those of skill in the art will be familiar with creation of myriad different nanoparticles of varying composition. For example, the nanoparticles can comprise metal (e.g., gold, silver, copper, aluminum, or platinum), plastic (e.g., polystyrene), a semiconductor material (e.g., CdSe, CdS, or CdSe coated with ZnS) or a magnetic material (e.g., ferromagnetite). Other nanoparticles herein can comprise one or more of: $ZnS$, $ZnO$, $TiO_2$, $Ag$, $AgI$, $AgBr$, $HgI_2$, $PbS$, $PbSe$, $ZnTe$, $CdTe$, and the like. Those of skill will also be familiar with various modifications (e.g., via thiol groups, etc.) of both nanoparticles and nucleotides to allow their attachment. Highly homogeneous particles, e.g., silver nanoclusters such as those with precise atomic numbers can also be used. The particles can also be used as scattering centers, detecting the back or forward scattering signal.

The size of the nanoparticle employed as a light scattering or light occluding framework in a given detectable construct of the invention can also range, varying from as large (or larger) than the size of the enzyme being assayed, to as small as a quantum dot. Thus, the nanoparticle frameworks can be <1 nm, 1 nm, 5 nm, 10 nm, 15 nm, 20 nm, 25 nm, 30 nm, 40 nm, 50 nm, 100 nm, or larger in diameter.

Methods of making metal and other nanoparticles are well known in the art. *See,* e.g., Schmid, G. (ed.) Clusters and Colloids, VCH, Weinheim, 1994; Hayat, M. A. (ed.) Colloidal Gold: Principles, Methods, and Applications, Academic Press, San Diego, 1991; Massart, IEEE Transactions On Magnetics, 1981, 17:1247+; Ahmadi, et al., Science, 1996, 272:1924+; Henglein, et al., J. Phys. Chem., 1995, 99:14129+; Curtis, et al., Angew. Chem. Int. Ed. Engl., 1988, 27:1530+; Weller, Angew. Chem. Int. Ed. Engl., 1993, 32:41+; Henglein, Top. Curr. Chem., 1988, 143:113+; Henglein, Chem. Rev., 1989, 89:1861+; Brus, Appl. Phys. A., 1991, 53:465+; Wang, J. Phys. Chem., 1991, 95:525+; Olshavsky, et al., J. Am. Chem. Soc., 1990, 112:9438+; and Ushida, et al., J. Phys. Chem., 1992, 95:5382+.

Either the nanoparticle frameworks, the nucleotides, or both are optionally functionalized in order to attach the nucleotides and the nanoparticles. Again, those of skill in the art will be familiar with such modifications. For instance, nucleotides herein are optionally functionalized with alkanethiols at their 3'-termini or 5'-termini (e.g., to attach to gold nanoparticles). *See* Whitesides, Proceedings of the Robert A. Welch Foundation 39th Conference On Chemical Research Nanophase Chemistry, Houston, Tex., pages 109-121 (1995) and Mucic, et al. Chem. Commun., 1966, 555-557. Functionalization via alkanethiol is also optionally used to attach nucleotides to other metal, semiconductor or magnetic nanoparticles. Additional functional groups used in attaching nucleotides to nanoparticles can include, e.g., phosphorothioate groups *(see,* e.g., U.S. Pat. No. 5,472,881), substituted alkylsiloxanes *(see,* e.g. Burwell, Chemical Technology, 1974, 4:370-377, Matteucci, J. Am. Chem. Soc., 1981, 103:3185-3191 (1981), and Grabar, et al., Anal. Chem., 67:735-743. Nucleotides terminated with a 5' thionucleoside or a 3' thionucleoside can also be used for attaching nucleotides/oligonucleotides to solid nanoparticles. *See also* Nuzzo, et al., J. Am. Chem. Soc., 1987, 109:2358; Allara, Langmuir, 1985, 1:45; Allara, Colloid Interface Sci., 1974, 49:410-421; Iler, The Chemistry Of Silica, Chapter 6, (Wiley 1979); Timmons, J. Phys. Chem., 1965, 69:984-990; and Soriaga, J. Am. Chem. Soc., 1982, 104:3937.

Further guidance of combinations of nanoparticles and nucleotides can be found in, e.g., U.S. Pat. Nos. 6,979,729 to Sperling et al.; 6,387,626 to Shi et al.; and 6,136,962 to Shi et al.; and 7,208,587 to Mirkin et al..

LABEL MOIETIES

In some embodiments, the detectable constructs of the invention further comprises at least one detectable label coupled to the framework and/or one or more member ligands; optionally, a plurality of labels are associated with the detectable construct.

In some embodiments, the one or more detectable labels are fluorescent labels. The members of the plurality of fluorescent labels can be the same fluorophore species or different fluorophores. An additional benefit to placing more than one fluorophore on a ligand-conjugated construct is that two or more types of fluorophores can be associated with the detectable construct, the combination of which would create new "colors" with which to uniquely identify the construct and associated ligand.

For example, the disclosure provides a set of four nucleotide-bearing constructs that can be differentiated using only two fluorophores. In the exemplary embodiment, nucleotide A is releasably coupled to a construct bearing, for example, twelve "green" fluors; nucleotide T is releasably coupled to a construct bearing twelve "red" fluors; nucleotide C is releasably coupled to a construct bearing eight "green" and four "red" fluors; while nucleotide G is releasably coupled to a construct bearing four "green" and eight "red" fluors. Each of these four combinations will have a unique spectral signature. In the methods and systems utilizing "green" and "red" fluors that are spectrally close together, only a single excitation laser need be provided for detection purposes. In addition, a smaller spectral window is analyzed, thus decreasing the number of camera pixels associated with each detection volume (e.g., ZMW), thus allowing for and/or increasing multiplex capability.

The above provides one exemplary embodiment; different quantities and/or ratios of the two fluorophores can be used to generate similarly distinguishable assay results. Fluorophores of varying excitation and emission frequencies are known in the art; one of skill would readily be able to select pairs of fluorophores and combinations other than those provided herein without undue experimentation.

Typically, the one or more label is associate with the framework portion of the construct (e.g., the label remains with the construct upon release of the ligand). In the above embodiments comprising a dendrimeric framework, the detectable label is optionally coupled to the double-stranded portion of the dendrimeric composition. Alternatively, the label is optionally associated with one or more single-stranded arms of the dendrimeric composition, e.g., via complementary binding. In embodiments comprising a circular nucleic acid framework, the label is optionally coupled to a double-stranded portion of the circular nucleic acid molecule.

In some of the methods of the present disclosure (such as described above for the two fluorophore system), more than one detectable construct is provided, wherein each construct has a different species of ligand associated therewith. In particular, for embodiments in which the enzyme is a polymerase, the methods provide four distinguishable detectable constructs, one for each nucleotide ligand. Preferably, each species of ligand comprising the plurality of ligands has a different detectable construct (e.g., different metal, magnetic, or light occluding particles), or different detectable labels or combination of detectable labels. The member labels, when present, are optionally coupled to framework (or, in some embodiments, the ligand) via a linker molecule.

The relative positions of the ligands and optional labels along the framework can vary from embodiment to embodiment. In some compositions, the member labels are coupled within a first region of the framework, and the ligands are coupled at a second region of the framework, positioned distal from the first region. In other embodiments, the labels and ligands are alternated spatially. The alternating labels and ligands can be sequestered to a specific portion of the framework, or they can be evenly distributed or randomly distributed along the framework.

The detectable construct can comprise more than one type or species of label. For example, in some embodiments, the plurality of labels comprises at least two species of fluorescent labels associated with the labeled construct. Optionally, the members of the two species of fluorescent labels are positioned proximal to one another, thereby enabling fluorescence resonance energy transfer (FRET).

As noted herein, the methods of the invention include providing a detectable construct. In some embodiments of the methods, providing the construct involves providing a first construct comprising one or more members of a first species of ligand, and providing a second construct comprising one or more members of a second species of ligand. In additional embodiments, four detectable constructs bearing four different species of ligand are provided, each construct having a plurality of the specified ligand species associated therewith. The step of detecting the construct includes distinguishing among the species of ligand. In a preferred embodiment, the enzyme comprises a polymerase and the ligands comprise one or more nucleotide or nucleotide analog. Each species of nucleotide or nucleotide analog is bourn by a detectable construct and are detectable (and thus distinguishable) from one another either in the framework, or an attached label or plurality of labels.

While the methods and compositions provided herein are not limited to a specific assay configuration, in a preferred embodiment, the detection volume proximal to the substrate surface comprises a zero mode waveguide.

## PROTECTIVE LAYERS

Optionally, the substrates provided in the methods and systems described herein further include a surface treatment, e.g., a protective layer or coating in contact with the substrate surface. The protective layer acts, e.g., as a shield from wet environments and can provide the substrate surface with some protection from liquids e.g., such as those involved in the enzyme-ligand interactions. The thickness of the protective layer can range from a few nanometers in depth to up to about 100 nm. Preferably, the protective layer is applied to the substrate surface prior to attachment of the enzyme; optionally, the protective layer provides one or more reactive groups for use in the attachment chemistries.

Compositions that can be used as a protective layer in the claims invention include, but are not limited to, those provided in US Patent publication numbers 2007-0314128 (to Korlach, titled "UNIFORM SURFACES FOR HYBRID MATERIAL SUBSTRATE AND METHODS FOR MAKING AND USING SAME") and 2008-0050747 (to Korlach and Turner, titled "ARTICLES HAVING LOCALIZED MOLECULES DISPOSED THEREON AND METHODS OF PRODUCING AND USING SAME).

## 3 DYE, 4 COLOR SEQUENCING DETECTION STRATEGIES

In some situations, problems can arise with excitation and independent detection of four unique fluorophores, or FRET pairs, during four color detection in single molecule sequencing. Such problem can arise, in part, from the overlap between laser excitation and fluorophore emission wavelengths and broad emission spectra of some fluorophores. Typically, the issues of spectral overlap can be addressed through use of appropriate filters in the optical train of the detection system. As will be appreciated, there is also a potential problem using FRET-pairs if there is poor energy transfer between the donor and acceptor. Such poor energy transfer can result in missed calls of nucleotides and miss-assignment of nucleotides when a strand is being read.

The instant embodiment corrects the problem of spectral overlap, which can occur through use of four unique fluorophores, by using only three fluorophores. The three fluorophores are selected so that they are easily separable with respect to excitation and emission (such as excitation wavelengths of 488, 568, and 647 nm). To perform four color sequencing with a three dye system, the three fluorophores are used alone while the two most spectrally isolated and non-interacting ones (e.g., 488 and 647 in the above illustration) are combined for the fourth base. This labeling strategy does not depend upon FRET, but instead uses a two-color signal associated with a given base. In particular, the detection of the fourth base (488-647) is indicated when there is signal coincidence in the 488 and 647 signals. When both signals start and/or stop at the same time, it indicates the presence of the fourth base.

It will be appreciated that the embodiment is not limited by particular types or identities of fluorophores to be used as long as the above excitation/emission criteria are followed (e.g., use of the two most spectrally isolated for the fourth nucleotide). In addition, it will be appreciated that a variety of two color combinations could be used on one, two, three or all four or more bases used in a given reaction, to provide an encoded signal associated with each reaction. Also, the current embodiment is not limited by particular methods of coincident detection.

## ADDITIONAL SYSTEM/APPARATUS DETAILS

The systems and apparatus of the invention can include optical detection systems (typically in those embodiments utilizing fluorescence or optical based systems) that include one or more of excitation light sources, detectors, and optical trains for transmitting excitation light to, and signal events from, the substrates or reaction vessels incorporating the analytical reactions of the invention. Examples of such systems include those described in Published U.S. Patent Application No. 2007-0036511, and U.S.7.995,202 (US Patent Application No. 11/704,689). The systems also optionally include additional features such as fluid handling elements for moving reagents into contact with one another or with the surfaces of the invention, robotic elements for moving samples or surfaces, and/or the like.

Laboratory systems of the invention optionally perform, e.g., repetitive fluid handling operations (e.g., pipetting) for transferring material to or from reagent storage systems that comprise samples of interest, such as microtiter trays, ZMWs, or the like. Similarly, the systems manipulate, e.g., microtiter trays, microfluidic devices, ZMWs or other components that constitute reagents, surfaces or compositions of the invention and/or that control any of a variety of environmental conditions such as temperature, exposure to light or air, and the like. Thus, systems of the invention can include standard sample handling features, e.g., by incorporating conventional robotics or microfluidic implementations. For example, a variety of automated systems components are available from Caliper Life Sciences Corporation (Hopkinton, MA), which utilize conventional robotics, e.g., for Zymate™ systems, as well as a variety of microfluidic implementations. For example, the LabMicrofluidic Device® high throughput screening system (HTS) is provided by Caliper Technologies, and the Bioanalyzer using LabChip™ technology is also provided by Caliper Technologies Corp and Agilent. Similarly, the common ORCA® robot, which is used in a variety of laboratory systems, e.g., for microtiter tray manipulation, is also commercially available, e.g., from Beckman Coulter, Inc. (Fullerton, CA).

Detection optics can be coupled to cameras, digital processing apparatus, or the like, to record and analyze signals detected in the various systems herein. Components can include a microscope, a CCD, a phototube, a photodiode, an LCD, a scintillation counter, film for recording signals, and the like. A variety of commercially available peripheral equipment and software is available for digitizing, storing and analyzing a digitized video or digitized optical image, e.g., using PC (Intel x86 or pentium chip-compatible DOS™, OS2™ WINDOWS™, WINDOWS NT™ or WINDOWS95™ based machines), MACINTOSH™, LINUX, or UNIX based (e.g., SUN™ work station) computers or digital appliances. Computers and digital appliances can include software for analyzing and perfecting signal interpretation. This can typically include standard application software such as spreadsheet or database software for storing signal information. However, systems of the invention can also include statistical analysis software to interpret signal data. For example, Partek Incorporated (St. Peters, MO.; on the World Wide Web at partek(dot)com) provides software for pattern recognition (e.g., which provide Partek Pro 2000 Pattern Recognition Software) which can be applied to signal interpretation and analysis. Computers/digital appliances also optionally include, or are operably coupled to, user viewable display systems (monitors, CRTs, printouts, etc.), printers to print data relating to signal information, peripherals such as magnetic or optical storage drives, and user input devices (keyboards, microphones, pointing devices), and the like. Detection components for non-optical based embodiments, e.g., electromagnetic based embodiments, as well as appropriate computer software for interpretation, storage, and display of non-optical data are also available and can be included in the systems herein.

Attaching and orienting enzymes to substrates

The ability to couple active enzymes to surfaces for readout of an assay such as a sequencing reaction is useful in a variety of settings. For example, enzyme activity can be measured in a solid phase format by binding the enzyme to a surface and performing the relevant assay. The ability to bind the enzyme to the surface has several advantages, including, but not limited to: the ability to purify, capture and assess enzyme reactions on a single surface; the ability to re-use the enzyme by washing ligand and reagents off of the solid phase between uses; the ability to format bound enzymes into a spatially defined set of reactions by selecting where and how the enzyme is bound onto the solid phase, facilitating monitoring of the reactions (e.g., using available arrays or ZMWs); the ability to perform and detect single-molecule reactions at defined sites on the substrate (thereby reducing reagent consumption); the ability to monitor multiple different enzymes on a single surface to provide a simple readout of multiple enzyme reactions at once, e.g., in biosensor applications, and many others.

Enzymes can be attached and oriented on a surface by controlling coupling of the enzyme to the surface. Examples of approaches for controllably coupling enzymes to a surface while retaining activity, e.g., by controlling the orientation of the enzyme and the distance of the enzyme from the surface are found, e.g., in US 2010/0260465 (U.S. Pat. Appl. No. 11/645,135).

Further details regarding orienting and coupling polymerases to surfaces so that activity is retained are found in US 2010/0260465 (U.S. Pat. Appl. No. 11/645,135).

One preferred class of enzymes in the various embodiments herein that can be fixed to a surface are DNA polymerases. For a review of polymerases, *see,* e.g., Hubscher, et al. (2002) EUKARYOTIC DNA POLYMERASES Annual Review of Biochemistry Vol. 71: 133-163; Alba (2001) "Protein Family Review: Replicative DNA Polymerases" Genome Biology 2(1): reviews 3002.1-3002.4; and Steitz (1999) "DNA polymerases: structural diversity and common mechanisms," J Biol Chem. 274:17395-17398.

Enzymes can conveniently be coupled to a surface by coupling the enzyme through an available artificial coupling domain, e.g., using any available coupling chemistry of interest. Exemplary coupling domains (which can be coupled to the enzyme, e.g., as an in frame fusion domain or as a chemically coupled domain) include any of: an added recombinant dimer enzyme or portion or domain of the enzyme, a large extraneous polypeptide domain, a polyhistidine tag, a HIS-6 tag, a biotin, an avidin sequence, a GST sequence, a glutathione, a AviTag sequence, an S tag, an antibody, an antibody domain, an antibody fragment, an antigen, a receptor, a receptor domain, a receptor fragment, a ligand, a dye, an acceptor, a quencher, and/or a combination thereof of any of the above.

Surfaces

The surfaces to which enzymes are bound can present a solid or semi-solid surface for any of a variety of linking chemistries that permit coupling of the enzyme to the surface. A wide variety of organic and inorganic materials, both natural and synthetic may be employed as the material for the surface in the various embodiments herein. Illustrative organic materials include, e.g., polymers such as polyethylene, polypropylene, poly(4-methylbutene), polystyrene, polymethylmethacrylate (PMMA), poly(ethylene terephthalate), rayon, nylon, poly(vinyl butyrate), polyvinylidene difluoride (PVDF), silicones, polyformaldehyde, cellulose, cellulose acetate, nitrocellulose, and the like. Other materials that can be employed as the surfaces or components thereof, include papers, ceramics, glass, metals, metalloids, semiconductive materials, cements, or the like. Glass represents one preferred embodiment. In addition, substances that form gels, such as proteins (e.g., gelatins), lipopolysaccharides, silicates, and agarose are also optionally used, or can be used as coatings on other (rigid, e.g., glass) surfaces.

In several embodiments herein, the solid surface is a planar, substantially planar, or curved surface such as an array chip, a wall of an enzymatic reaction vessel such as a sequencing or amplification chamber, a ZMW or the like.

In particular embodiments, surfaces can comprise silicate elements (e.g., glass or silicate surfaces). A variety of silicon-based molecules appropriate for functionalizing such surfaces is commercially available. *See,* for example, Silicon Compounds Registry and Review, United Chemical Technologies, Bristol, PA. Additionally, the art in this area is very well developed and those of skill will be able to choose an appropriate molecule for a given purpose. Appropriate molecules can be purchased commercially, synthesized *de novo,* or can be formed by modifying an available molecule to produce one having the desired structure and/or characteristics.

Linking groups can also be incorporated into the enzymes to aid in enzyme attachment. Such groups can have any of a range of structures, substituents and substitution patterns. They can, for example, be derivatized with nitrogen, oxygen and/or sulfur containing groups which are pendent from, or integral to, the linker group backbone. Examples include, polyethers, polyacids (polyacrylic acid, polylactic acid), polyols (e.g., glycerol), polyamines (e.g., spermine, spermidine) and molecules having more than one nitrogen, oxygen and/or sulfur moiety (e.g., 1,3-diamino-2-propanol, taurine). *See,* for example, Sandler, et al. (1983) Organic Functional Group Preparations 2nd Ed., Academic Press, Inc. San Diego. A wide range of mono-, di- and bis-functionalized poly(ethyleneglycol) molecules are commercially available. Coupling moieties to surfaces can also be done via light-controllable methods, i.e., utilize photo-reactive chemistries.

Enzymes bound to solid surfaces as described above can be formatted into sets/libraries of components. The precise physical layout of these libraries is at the discretion of the practitioner. One can conveniently utilize gridded arrays of library members (e.g., individual bound enzymes, or blocks of enzyme bound at fixed locations), e.g., on a glass or polymer surface, or formatted in a microtiter dish or other reaction vessel, or even dried on a substrate such as a membrane. However, other layout arrangements are also appropriate, including those in which the library members are stored in separate locations that are accessed by one or more access control elements (e.g., that comprise a database of library member locations). The library format can be accessible by conventional robotics or microfluidic devices, or a combination thereof.

In addition to libraries that comprise liquid phase components, libraries can also simply comprise solid phase arrays of enzymes (e.g., that can have liquid phase reagents added to them during operation). These arrays fix enzymes in a spatially accessible pattern (e.g., a grid of rows and columns) onto a solid substrate such as a membrane (e.g., nylon or nitrocellulose), a polymer or ceramic surface, a glass or modified silica surface, a metal surface, or the like. The libraries can also be formatted on a ZMW.

## Claims

1.  A method of monitoring a single molecule real-time enzymatic reaction between an enzyme and a member ligand of a plurality of ligands, the method comprising:

    providing a substrate comprising a substrate surface, a zero mode waveguide (ZMW) detection volume proximal to the substrate surface, and a single molecule of an enzyme positioned within the ZMW detection volume and bound to or associated with the substrate surface, wherein the enzyme comprises a polymerase;
    contacting the enzyme with a detectable construct comprising a labelled DNA dendrimer or a labelled circular DNA framework, wherein multiple ligands specific for the enzyme are removably coupled to the framework and wherein the ligands comprise one or more nucleotides or nucleotide analogs; and
    detecting the construct while the enzyme and a member ligand of the multiple ligands are interacting, thereby monitoring the enzymatic reaction.

2.  A system for monitoring a single molecule real-time enzymatic reaction, the system comprising:

    a substrate comprising a substrate surface and a ZMW detection volume proximal to the substrate surface, wherein the enzyme comprises a polymerase;
    a single molecule of an enzyme, which enzyme is positioned within the ZMW detection volume and bound to or associated with the substrate surface;
    a detectable construct comprising a labelled DNA dendrimer or a labelled circular DNA framework, wherein multiple ligands specific for the enzyme are removably coupled to the framework and wherein the ligands comprise one or more nucleotides or nucleotide analogs; and
    a detector functionally coupled to the substrate surface and capable of detecting the construct when the construct is in proximity of the enzyme.

3.  The method of claim 1 or the system of claim 2, wherein the framework comprises a labeled circular nucleic acid species.

4.  The method of claim 1 or the system of claim 2, wherein the framework comprises an occluding and/or light scattering moiety, and wherein detecting the construct comprises monitoring a light transmission past or through the substrate surface and/or monitoring light scattering away from the substrate surface.

5.  The method or system of claim 4, wherein the occluding and/or light scattering moiety comprises a metal nanoparticle, a plastic nanoparticle, a glass nanoparticle, or a semiconductor material nanoparticle.

6. The method of claim 1 or the system of claim 2, wherein the framework comprises a fluorescent particle to which at least two ligands of a given type are coupled.

7. The method or system of claim 6, wherein the fluorescent particle comprises a quantum dot, a nanoparticle, or a nanobead.

8. The method of claim 1 or the system of claim 2, wherein the detectable construct further comprises at least one detectable label attached to the framework and/or attached to one or more member ligand(s).

9. The method or system of claim 8, wherein the at least one detectable label comprises a plurality of labels coupled to the framework.

10. The method or system of claim 9, wherein each species of ligand comprising the multiple ligands comprises a different detectable label or combination of detectable labels; or
the plurality of labels are coupled to the framework by a linker molecule; or
the plurality of labels are coupled within a first region of the framework, and member ligands of the multiple ligands are coupled at a second region of the framework, wherein the second region of the framework is distal from the first region; or
member labels of the plurality of labels are spatially alternated with member ligands of the plurality of ligands on the nucleic acid framework; or
the plurality of labels comprises at least two species of fluorescent labels, and
wherein members of the two species of fluorescent labels are positioned proximal to one another thereby enabling fluorescence resonance energy transfer (FRET).


**Patentansprüche**

1. Verfahren zum Überwachen einer enzymatischen Einzelmolekül-Echtzeit-Reaktion zwischen einem Enzym und einem Ligandenelement aus einer Vielzahl von Liganden, wobei das Verfahren Folgendes umfasst:

Bereitstellen eines Substrats, umfassend eine Substratoberfläche, ein Nullmoden-Wellenleiter- (ZMW-) Detektionsvolumen in der Nähe der Substratoberfläche und ein Einzelmolekül eines Enzyms, das innerhalb des ZMW-Detektionsvolumens positioniert ist und an die Substratoberfläche gebunden oder damit assoziiert ist, wobei das Enzym eine Polymerase umfasst;
Kontaktieren des Enzyms mit einem detektierbaren Konstrukt, umfassend ein markiertes DNA-Dendrimer oder ein markiertes kreisförmiges DNA-Gerüst, wobei mehrere Liganden, die für das Enzym spezifisch sind, entfernbar an das Gerüst gekuppelt sind und wobei die Liganden ein oder mehrere Nucleotide oder NucleotidAnaloga umfassen; und
Detektieren des Konstrukts während das Enzym und ein Ligandenelement aus mehreren Liganden wechselwirken, wodurch die enzymatische Reaktion überwacht wird.

2. System zum Überwachen einer enzymatischen Einzelmolekül-Echtzeit-Reaktion, wobei das System Folgendes umfasst:

ein Substrat, umfassend eine Substratoberfläche und ein ZMW-Detektionsvolumen in der Nähe der Substratoberfläche, wobei das Enzym eine Polymerase umfasst;
ein Einzelmolekül eines Enzyms, wobei das Enzym innerhalb des ZMW-Detektionsvolumens positioniert und an die Substratoberfläche gebunden oder damit assoziiert ist;
ein detektierbares Konstrukt, umfassend ein markiertes DNA-Dendrimer oder ein markiertes kreisförmiges DNA-Gerüst, wobei mehrere Liganden, die für das Enzym spezifisch sind, entfernbar an das Gerüst gekuppelt sind und wobei die Liganden ein oder mehrere Nucleotide oder Nucleotidanaloga umfassen; und
einen Detektor, der funktionell an die Substratoberfläche gekuppelt ist und dazu in der Lage ist, das Konstrukt zu detektieren, wenn sich das Konstrukt in der Nähe des Enzyms befindet.

3. Verfahren nach Anspruch 1 oder System nach Anspruch 2, wobei das Gerüst eine markierte kreisförmige Nucleinsäurespezies umfasst.

**4.** Verfahren nach Anspruch 1 oder System nach Anspruch 2, wobei das Gerüst eine verschließende und/oder lichtstreuende Gruppierung umfasst und wobei das Detektieren des Konstrukts das Überwachen einer Lichttransmission vorbei an oder durch die Substratoberfläche und/oder das Überwachen einer Lichtstreuung weg von der Substratoberfläche umfasst.

**5.** Verfahren oder System nach Anspruch 4, wobei die verschließende und/oder lichtstreuende Gruppierung ein Metallnanoteilchen, ein Kunststoffnanoteilchen, ein Glasnanoteilchen oder ein Nanoteilchen aus Halbleitermaterial umfasst.

**6.** Verfahren nach Anspruch 1 oder System nach Anspruch 2, wobei das Gerüst ein fluoreszierendes Teilchen umfasst, an das zumindest zwei Liganden eines gegebenen Typs gekuppelt sind.

**7.** Verfahren oder System nach Anspruch 6, wobei das fluoreszierende Teilchen einen Quantenpunkt, ein Nanoteilchen oder ein Nanokügelchen umfasst.

**8.** Verfahren nach Anspruch 1 oder System nach Anspruch 2, wobei das detektierbare Konstrukt weiters zumindest eine detektierbare Markierung umfasst, die an das Gerüst und/oder an ein oder mehrere Ligandenelemente gebunden ist.

**9.** Verfahren oder System nach Anspruch 8, wobei die zumindest eine detektierbare Markierung eine Vielzahl von an das Gerüst gekuppelten Markierungen umfasst.

**10.** Verfahren oder System nach Anspruch 9, wobei jede Spezies von Ligand, die in den mehreren Liganden umfasst ist, eine andere detektierbare Markierung oder eine Kombination von detektierbaren Markierungen umfasst; oder die Vielzahl von Markierungen über ein Linkermolekül an das Gerüst gekuppelt ist; oder die Vielzahl von Markierungen innerhalb einer ersten Region des Gerüsts gekuppelt ist und Ligandenelemente aus den mehreren Liganden an einer zweiten Region des Gerüsts gekuppelt sind, wobei die zweite Region des Gerüsts sich distal von der ersten Region befindet; oder sich Markierungselemente aus der Vielzahl von Markierungen räumlich mit Ligandenelementen aus der Vielzahl von Liganden auf dem Nucleinsäuregerüst abwechseln; oder die Vielzahl von Markierungen zumindest zwei Spezies von fluoreszierenden Markierungen umfasst und wobei Elemente der zwei Spezies von fluoreszierenden Markierungen in der Nähe voneinander positioniert sind, wodurch ein Fluoreszenzresonanzenergietransfer (FRET) ermöglicht wird.

**Revendications**

**1.** Procédé pour suivre une réaction enzymatique en temps réel d'une molécule unique entre une enzyme et un élément ligand d'une pluralité de ligands, le procédé comprenant les étapes consistant à :

se procurer un substrat comprenant une surface de substrat, un volume de détection de guide d'ondes en mode zéro (ZMW) proximal à la surface de substrat, et une molécule unique d'une enzyme placée dans le volume de détection de ZMW et liée ou associée à la surface de substrat, où l'enzyme comprend une polymérase ;
mettre en contact l'enzyme avec une construction détectable comprenant un dendrimère d'ADN marqué ou une ossature d'ADN circulaire marqué, où de multiples ligands spécifiques de l'enzyme sont couplés de façon séparable à l'ossature, et où les ligands comprennent un ou plusieurs nucléotides ou analogues de nucléotides ; et
détecter la construction pendant que l'enzyme et un élément ligand des multiples ligands interagissent, permettant ainsi de suivre la réaction enzymatique.

**2.** Système pour suivre une réaction enzymatique en temps réel d'une molécule unique, le système comprenant :

un substrat comprenant une surface de substrat et un volume de détection de ZMW proximal à la surface de substrat, où l'enzyme comprend une polymérase ;
une molécule unique d'une enzyme, laquelle enzyme est placée dans le volume de détection de ZMW et liée ou associée à la surface de substrat ;
une construction détectable comprenant un dendrimère d'ADN marqué ou une ossature d'ADN circulaire marqué, où de multiples ligands spécifiques de l'enzyme sont couplés de façon séparable à l'ossature et où les ligands comprennent un ou plusieurs nucléotides ou analogues de nucléotides ; et
un détecteur fonctionnellement couplé à la surface de substrat et capable de détecter la construction lorsque la construction est à proximité de l'enzyme.

3. Procédé selon la revendication 1 ou système selon la revendication 2, dans lequel l'ossature comprend une espèce d'acide nucléique circulaire marqué.

4. Procédé selon la revendication 1 ou système selon la revendication 2, dans lequel l'ossature comprend une fraction occultant et/ou diffusant la lumière, et dans lequel la détection de la construction consiste à suivre une transmission lumineuse devant ou à travers la surface de substrat et/ou à suivre la diffusion lumineuse loin de la surface de substrat.

5. Procédé ou système selon la revendication 4, dans lequel la fraction occultant et/ou diffusant la lumière comprend une nanoparticule de métal, une nanoparticule de plastique, une nanoparticule de verre, ou une nanoparticule de matériau semi-conducteur.

6. Procédé selon la revendication 1 ou système selon la revendication 2, dans lequel l'ossature comprend une particule fluorescente à laquelle au moins deux ligands d'un type donné sont couplés.

7. Procédé ou système selon la revendication 6, dans lequel la particule fluorescente comprend un point quantique, une nanoparticule, ou une nanobille.

8. Procédé selon la revendication 1 ou système selon la revendication 2, dans lequel la construction détectable comprend en outre au moins un marqueur détectable fixé à l'ossature et/ou fixé à un ou à plusieurs éléments ligands.

9. Procédé ou système selon la revendication 8, dans lequel ledit au moins un marqueur détectable comprend une pluralité de marqueurs couplés à l'ossature.

10. Procédé ou système selon la revendication 9, dans lequel chaque espèce de ligand constituant les multiples ligands comprend un marqueur détectable différent ou une combinaison de marqueurs détectables ; ou
la pluralité de marqueurs sont couplés à l'ossature par une molécule de lieur ; ou
la pluralité de marqueurs sont couplés au sein d'une première région de l'ossature, et des éléments ligands des multiples ligands sont couplés au niveau d'une seconde région de l'ossature, cette seconde région de l'ossature étant distale par rapport à la première région ; ou
des éléments marqueurs de la pluralité de marqueurs sont spatialement alternés avec des éléments ligands de la pluralité de ligands sur l'ossature d'acide nucléique ; ou
la pluralité de marqueurs comprend au moins deux espèces de marqueurs fluorescents, lesquels membres des deux espèces de marqueurs fluorescents sont placés de façon proximale les uns par rapport aux autres, permettant ainsi un transfert d'énergie par résonance de fluorescence (FRET).

Fig. 1A

Fig. 1B

Fig. 1C

Fig. 1D

Fig. 1E

## REFERENCES CITED IN THE DESCRIPTION

### Patent documents cited in the description

- US 5982534 A
- US 6169289 B
- WO 2006044078 A
- WO 03014743 A
- US 6399335 B
- US 6917726 B
- US 7013054 B
- US 7033764 B
- US 7052847 B
- US 7056661 B
- US 7056676 B
- US 7181122 B
- US 20030044781 A
- US 6689565 B, Nikiforov
- US 6979729 B
- US 6387626 B
- US 6136962 A
- US 20040072231 A
- US 5472881 A
- US 7208587 B, Mirkin
- US 20070314128 A
- US 20080050747 A
- US 20070036511 A
- US 7995202 B
- US 11704689 B
- US 20100260465 A
- US 645135 A

### Non-patent literature cited in the description

- **LEVENE et al.** Zero-Mode Waveguides for Single Molecule Analysis at High Concentrations. *Science,* 2003, vol. 299, 682-686
- **ASLAN et al.** Metal-enhanced fluorescence: an emerging tool in biotechnology. *Current Opinion in Biotechnology,* 2005, vol. 16, 55-62
- **LAKOWICZ et al.** Intrinsic Fluorescence from DNA Can Be Enhanced by Metallic Particles. *Biochemical and Biophysical Research Communications,* 2001, vol. 286, 875-879
- **EID et al.** *Science,* 2009, vol. 323, 133-138
- **STRIEBEL et al.** *Exp. Mol. Path.,* 2004, vol. 77, 87-97
- **EID et al.** Real-Time DNA Sequencing from Single Polymerase Molecules. *Science,* 20 November 2008
- **LEVENE et al.** Zero-mode waveguides for single-molecule analysis at high concentrations. *Science,* 2003, vol. 299, 682-686
- **CZESLIK et al.** *Biophys. J,* 2003, vol. 84, 2533
- **DEHONG HU ; H. PETER LU.** *J. Phys. Chem. B,* 2003, vol. 107, 618
- **J. WENGER et al.** *Optics Express,* 2005, vol. 13, 7035
- **DEHONG HU ; PETER LU.** *J. Phys. Chem. B,* 2003, vol. 107, 618
- **NILSEN et al.** Dendritic Nucleic Acid Structures. *J. Theoretical Biology,* 1997, vol. 187, 273-284
- **WANG et al.** Dendritic Nucleic Acid Probes for DNA Biosensors. *JACS,* 1998, vol. 120, 8281-8282
- **HENG et al.** Characterization of light collection through a subwavelength aperture from a point source. *Optics Express,* 2006, vol. 14 (22), 10410-10425
- Clusters and Colloids. VCH, 1994
- Colloidal Gold: Principles, Methods, and Applications. Academic Press, 1991
- **MASSART.** *IEEE Transactions On Magnetics,* 1981, vol. 17, 1247
- **AHMADI et al.** *Science,* 1996, vol. 272, 1924
- **HENGLEIN et al.** *J. Phys. Chem.,* 1995, vol. 99, 14129
- **CURTIS et al.** *Angew. Chem. Int. Ed. Engl.,* 1988, vol. 27, 1530
- **WELLER.** *Angew. Chem. Int. Ed. Engl.,* 1993, vol. 32, 41
- **HENGLEIN.** *Top. Curr. Chem.,* 1988, vol. 143, 113
- **HENGLEIN.** *Chem. Rev.,* 1989, vol. 89, 1861
- **BRUS.** *Appl. Phys. A.,* 1991, vol. 53, 465
- **WANG.** *J. Phys. Chem.,* 1991, vol. 95, 525
- **OLSHAVSKY et al.** *J. Am. Chem. Soc.,* 1990, vol. 112, 9438
- **USHIDA et al.** *J. Phys. Chem.,* 1992, vol. 95, 5382
- **WHITESIDES.** *Proceedings of the Robert A. Welch Foundation 39th Conference On Chemical Research Nanophase Chemistry,* 1995, 109-121
- **MUCIC et al.** *Chem. Commun.,* vol. 1966, 555-557

- **BURWELL.** *Chemical Technology,* 1974, vol. 4, 370-377
- **MATTEUCCI.** *J. Am. Chem. Soc.,* 1981, vol. 103, 3185-3191
- **GRABAR et al.** *Anal. Chem.,* vol. 67, 735-743
- **NUZZO et al.** *J. Am. Chem. Soc.,* 1987, vol. 109, 2358
- **ALLARA.** *Langmuir,* 1985, vol. 1, 45
- **ALLARA.** *Colloid Interface Sci.,* 1974, vol. 49, 410-421
- **ILER.** The Chemistry Of Silica. Wiley, 1979
- **TIMMONS.** *J. Phys. Chem.,* 1965, vol. 69, 984-990
- **SORIAGA.** *J. Am. Chem. Soc.,* 1982, vol. 104, 3937
- **HUBSCHER et al.** EUKARYOTIC DNA POLYMERASES. *Annual Review of Biochemistry,* 2002, vol. 71, 133-163
- **ALBA.** Protein Family Review: Replicative DNA Polymerases. *Genome Biology,* 2001, vol. 2 (1), 3002.1-3002.4
- **STEITZ.** DNA polymerases: structural diversity and common mechanisms. *J Biol Chem,* 1999, vol. 274, 17395-17398
- Silicon Compounds Registry and Review. United Chemical Technologies
- **SANDLER et al.** Organic Functional Group Preparations. Academic Press, 1983